(19) Europäisches Patentamt
European Patent Office
Office européen des brevets

(11) Publication number: **0 024 096**
**B1**

# (12) EUROPEAN PATENT SPECIFICATION

(45) Date of publication of patent specification: **25.05.83**

(21) Application number: **80301723.5**

(22) Date of filing: **23.05.80**

(51) Int. Cl.$^3$: **C 07 F 1/08,**
C 07 C 147/14,
C 07 C 103/36,
C 07 C 65/00,
C 07 C 101/52,
C 07 C 149/20,
C 07 C 163/00,
A 61 K 31/30, A 61 K 31/60

## (54) Complexes of bivalent copper, methods of preparation thereof and compositions containing said complexes.

(30) Priority: **15.08.79 AU 41/79**

(43) Date of publication of application:
**25.02.81 Bulletin 81/8**

(45) Publication of the grant of the patent:
**25.05.83 Bulletin 83/21**

(84) Designated Contracting States:
**BE CH DE FR GB LI LU NL SE**

(56) References cited:
**EP - A - 0 002 341**

(73) Proprietor: **Boettcher, Barry**
**12 Mahogany Drive**
**New Lambton New South Wales, 2305 (AU)**

(73) Proprietor: **Walker, William Raymond**
**University of Newcastle**
**New South Wales, 2308 (AU)**

(73) Proprietor: **Whitehouse, Michael Wellesley**
**Department of Experimental Pathology Australian National University**
**Canberra A.C.T., 2600 (AU)**

(72) Inventor: **Boettcher, Barry**
**12 Mahogany Drive**
**New Lambton New South Wales, 2305 (AU)**
Inventor: **Walker, William Raymond**
**University of Newcastle**
**New South Wales, 2308 (AU)**
Inventor: **Whitehouse, Michael Wellesley**
**Department of Experimental Pathology Australian National University**
**Canberra A.C.T., 2600 (AU)**

(74) Representative: **Burnside, Michael et al,**
**c/o Michael Burnside & Partners 2 Serjeants' Inn**
**Fleet Street**
**London EC4Y 1HL (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

EP 0 024 096 B1

Complexes of bivalent copper, methods of preparation thereof and compositions containing sad complexes

This invention relates to novel anti-inflammatory copper complexes and to anti-inflammatory compositions and processes utilizing such complexes.

Many studies of the role of copper, and especlly complexes of copper, in the treatment of inflammatory diseases have been reported in recent years. One of the most recent studites describes the use of parenterally administered copper complexes, and particularly copper ZII) salicylate, in the treatment of rheumatic disease (Sorenson and Hangarter, Inflammation, *2*, 217—238 (1977)).

The prior art describes e wide variety of copper-salicylate complexes prepared by the reaction of inorganic copper (II) salts with salicyclic acid in aqueous solution. The specific complex formed in aqueous solution has been found to depend on the pH of the aqueous solution and both neutral complexes such as the pale blue crystalline bis (salicylato) copper (II) tetrahydrate

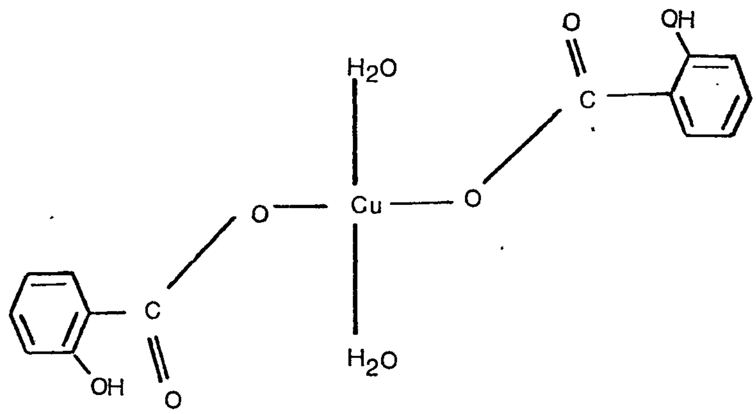

and anionic complexes such as the olive green sodium salicylatocuprate (II)

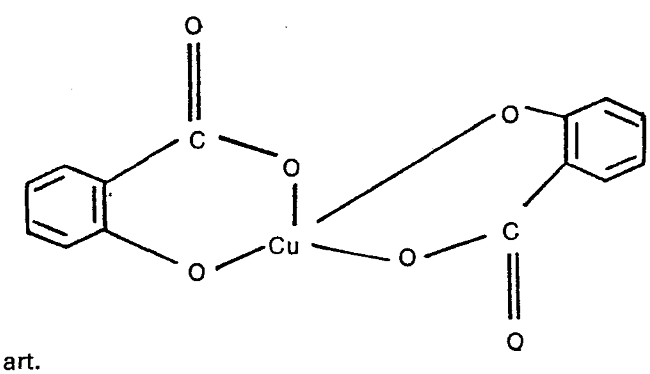

are known in the art.

In our prior European patent application no. 78300671.1 (Patent No. 0002341) we described the use of copper complexes prepared by the reaction of copper compounds and substituted benzoic acids in the presence of an alkanol.

Moreover, a copper salicylate-dimethyl formamide complex is disclosed by Suntsov and Popovitch [Akademia Nauk. SSR—Doklady Chemistry 179, 1352 (1968)]. No pharmaceutical use is, however, given for this complex.

It is an object of this invention to provide novel neutral copper complexes prepared by the reaction of copper compounds and substituted benzoic acids in the presence of dimethylsulphoxide or dimethylformamide. A further object of the invention is to provide a method for the treatment of inflammatory diseases of animals by the administration of compositions comprising as active ingredients the novel copper complexes of the invention.

Accordingly in one embodiment the invention provides neutral copper complexes of the formula $Cu[C_6H_4(X)COO]_2$ Y wherein Y represents dimethylsulphoxide or dimethylformamide, and wherein X can be located in any position and is OH, SH, SeH, $NH_2$ or

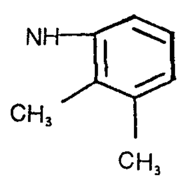

provided that, when X is OH, Y is not dimethylformamide.

This invention also provides a pharmaceutical composition comprising a neutral copper complex and a pharmaceutically acceptable carrier, characterized in that said neutral copper complex has the formula

$$Cu[C_6H_4(X)COO]_2\ Y$$

wherein Y represents dimethylsulphoxide or dimethylformamide, and wherein X can be located in any position and is OH, SH, SeH, $NH_2$ or

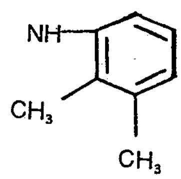

This invention also provides a neutral copper complex of the formula

$$Cu[C_6H_4(X)COO]_2\ Y$$

wherein Y represents dimethylsulphoxide or dimethylformamide, and wherein X can be located in any position and is OH, SH, SeH, $NH_2$ or

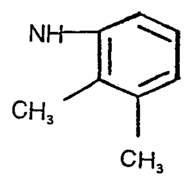

for use in treating inflammatory diseases.

Although in no way wishing to be bound by theory, as a result of physico-chemical studies the complexes of the invention are believed to have the following dimeric structure:

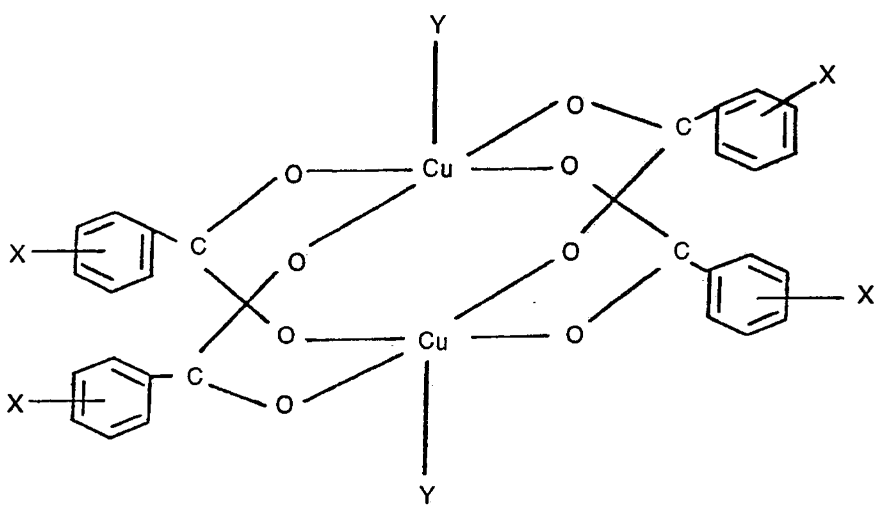

The preferred compound according to the invention is the copper salicylate complex which is believed to have the formula

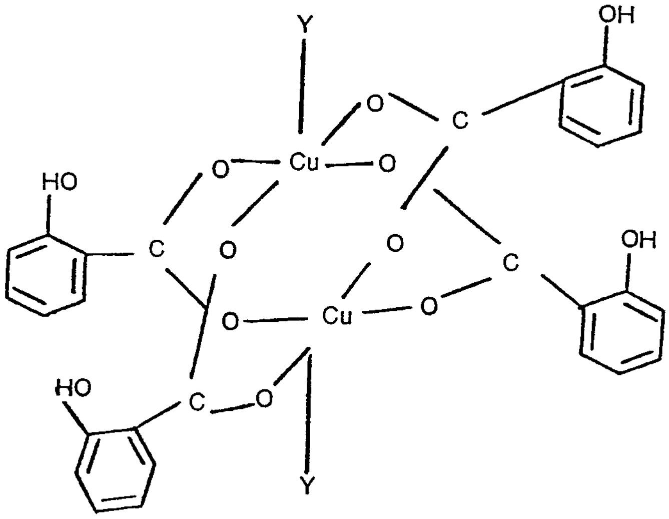

The compounds of the invention may be prepared by reacting inorganic copper (II) compounds, for example copper salts or cupric hydroxide, with the appropriate substituted benzoic acid in the presence of dimethylsulphoxide or dimethylformamide. For example, reaction of cupric hydroxide with salicylic acid and dimethylsulphoxide gives the complex of formula $Cu[C_6H_4(OH)COO]_2.\ (CH_3)_2SO$, hereinafter referred to by the formula $Cu[H\ Sal]_2$. D.M.S.O., which forms fine deep green crystals when crystallised from anhydrous dimethylsulphoxide containing excess (approx. 5M) salicylic acid.

The compounds of the invention have been found to be particularly useful in the treatment of inflammatory diseases in animals. Thus in a further embodiment the invention provides a process for the treatment of inflammatory diseases of animals which process comprises administering to said animals an effective amount of a compound of the invention as hereinbefore defined.

The compounds of the invention have proved particularly effective in alleviating the symptoms of inflammatory diseases such as rheumatic disease, arthritis and rheumatoid arthritis when applied topically to the animal.

Preferably the compounds of the invention are applied in the form of a composition comprising a compound of the invention in admixture with a pharmaceutically acceptable carrier. Therefore, in yet a further aspect the invention provides pharmaceutical anti-inflammatory compositions comprising a compound of the invention as hereinbefore defined and a pharmaceutically acceptable carrier therefor. The carrier preferably comprises an excess of the dimethylsulphoxide or dimethylformamide and an emollient such as glycerol.

Preferably the composition also contains a buffer for increasing the pH to prevent irritation by the salicylic acid; such buffers may include, for example, sodium acetate.

The compositions are preferably suitable for topical application to animals to be treated and therefore may be in the form of a gel, an ointment, a paste, a cream or a lotion. Compositions comprising a compound of the invention in solution are preferred as they are more efficient in perfusing the skin.

The amount of the compound of the invention employed in the compositions will depend to a large extent on the inflammatory condition being treated. However, as a general rule the compositions may comprise from 0.1 to 15% w/v of the copper compounds of the invention and preferably from 1 to 7% w/v.

As previously indicated the compounds of the invention have proved particularly useful in the alleviation of the symptoms of inflammatory disease when applied topically to the animal in the form of a pharmaceutical composition as hereinbefore defined. The compounds are believed to be efficacious in such treatments because of their ready penetration of the skin. Furthermore, the compounds are believed to offer particular advantages in the treatment of inflammatory diseases of humans because of the non-toxic nature of the compounds and evidence that the compounds are readily cleared from the treated animal as indicated by the limited duration of the anti-inflammatory effect of the compounds.

The compositions may comprise, in addition to one or more compounds of the invention, other pharmaceutically active ingredients including other anti-inflammatory agents and conventional pharmaceutical excipients known in the art.

The invention is now illustrated by but not limited to the following examples.

Example 1

Preparation of $Cu[C_6H_4(OH)COO]_2.\ (CH_3)_2SO$

Cupric hydroxide (1.0 g) and salicylic acid (5.0 g) were dissolved in dimethylsulphoxide (10 ml)

and heated on a water bath. The solution was refrigerated for 48 hours. Fine deep-green crystals were deposited, filtered off, washed with benzene and dried over $P_2O_5$ under vacuum.
Yield 0.5 g (Found: Cu, 15.5; C, 45.9; H, 4.0; S7.8;
$CuC_{16}H_{16}O_7S$ requires Cu, 15.4; C, 46.1; H, 4.3; S78%)

Example 2

Preparation of $Cu[C_6H_4(OH)COO]_2$. $C_3H_7NO$

Cupric hydroxide (1.0 g) and silicylic acid (5.0 g) were dissolved in N,N-dimethylformamide (12 ml) and heated on a hot water bath. After refrigerating the resultant solution for 24 hours, green crystals were deposited. These were filtered off, washed with benzene and dried over $P_2O_5$ under vacuum. Yield 0.43 g.
(Found: Cu 15.4; C 49.6; H 4.1; N 3.3% $CuC_{17}H_{17}O_7N$
requires Cu 15.5; C 49.7; H 4.2; N 3.4%).

The products of the invention are rapidly absorbed through the skin and are promising materials for the treatment of copper deficiency conditions, for example Menkes' syndrome in which copper cannot be metabolised from the gut, leading to mental retardation, albinism and central nervous system disorders.

The absorption of topically applied products of the invention is illustrated in the following example.

Example 3

A neutral copper salicylate-DMSO complex was prepared, as in Example 1 by mixing 1 g $Cu(OH)_2$ 7 g salicylic acid, 60 ml ethanol, 20 ml DMSO and 20 ml glycerol. 3 ml of the composition was rubbed on a human forearm and the concentration of salicylic acid measured in the serum. For comparison, the same concentration of salicylic acid in same vehicle was used. High pressure liquid chromatography was used for analysis.

| | | Conc$^n$. Test Preparation | $H_2$sal* Vehicle |
|---|---|---|---|
| Time *after* application | 2h | 0.4 | 0.7 |
| | 4h | 1.7 | 1.3 |
| | 6h | 1.3 | 0.8 |

*$\mu$g/ml serum.

**Claims**

1. Neutral copper complexes characterized in that they have the formula

$$Cu[C_6H_4(X)COO]_2\ Y$$

wherein Y represents dimethylsulphoxide or dimethylformamide, and wherein X can be located in any position and is OH, SH, SeH, $NH_2$ or

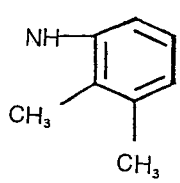

provided that, when X is OH, Y is not dimethylformamide.

2. Complexes according to claim 1 characterized in that they have the formula

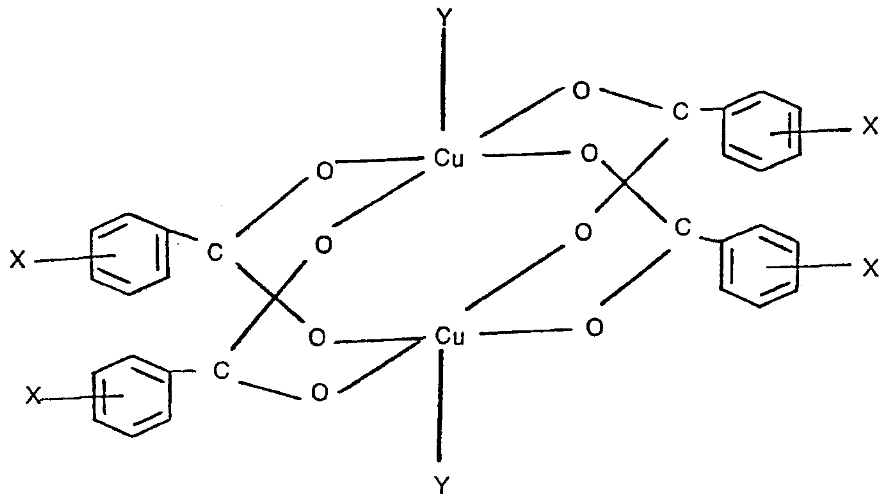

wherein Y and X are as defined in claim 1.

3. Complexes according to claim 1 or claim 2, characterized in that X is OH and Y is dimethylsulphoxide.

4. Complexes according to claim 2 characterized in that they have the formula

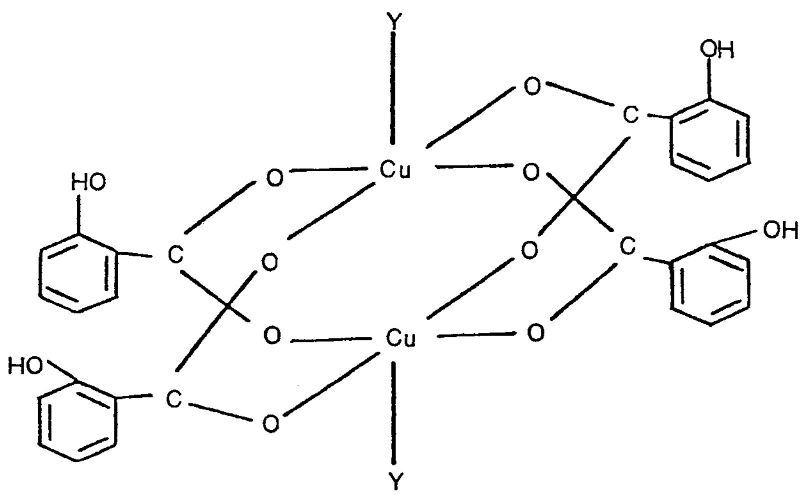

(II)

wherein Y is as defined in claim 2.

5. A method of preparing complexes as claimed in any of claims 1 to 4 characterized in that it comprises reacting an inorganic copper (II) compound with a substituted benzoic acid in the presence of dimethylsulphoxide or dimethylformamide.

6. A method according to claim 5 characterized in that the substituted benzoic acid is salicylic acid.

7. A method according to claim 5 or claim 6, characterized in that the copper (II) compound is a salt or cupric hydroxide.

8. A pharmaceutical composition comprising a neutral copper complex and a pharmaceutically acceptable carrier, characterized in that said neutral copper complex has the formula

$$Cu[C_6H_4(X)COO]_2\ Y$$

wherein Y represents dimethylsulphoxide or dimethylformamide, and wherein X can be located in any position and is OH, SH, SeH, $NH_2$ or

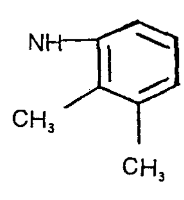

9. A composition according to claim 8, characterized in that the pharmaceutically acceptable carrier is a non-aqueous carrier.

10. A composition according to claim 8, characterized in that the carrier comprises dimethylsulphoxide or dimethylformamide; glycerol; and salicylic acid.

11. A composition according to any one of claims 8 to 10 which contains a buffer.

12. A composition according to any one of claims 8 to 11 characterized in that it contains 0.1 to 15% w/v of active ingredient.

13. A neutral copper complex of the formula

$$Cu[C_6H_4(X)COO]_2\ Y$$

wherein Y represents dimethylsulphoxide or dimethylformamide, and wherein X can be located in any position and is OH, SH, SeH, $NH_2$ or

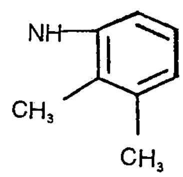

for use in treating inflammatory diseases.

**Patentansprüche**

1. Neutrale Kupferkomplexe, gekennzeichnet durch die Formel

$$Cu[C_6H_4(X)COO]_2\ Y$$

in der Y Dimethylsulfoxid oder Dimethylformamid ist, und in der X an beliebigen Positionen angeordnet sein kann und OH, SH, SeH, $NH_2$ oder

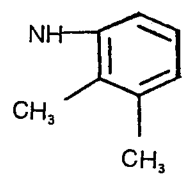

bedeutet, unter der Voraussetzung, daß, wenn X OH ist, Y nicht Dimethylformamid ist.

2. Komplexe nach Anspruch 1, gekennzeichnet durch die Formel

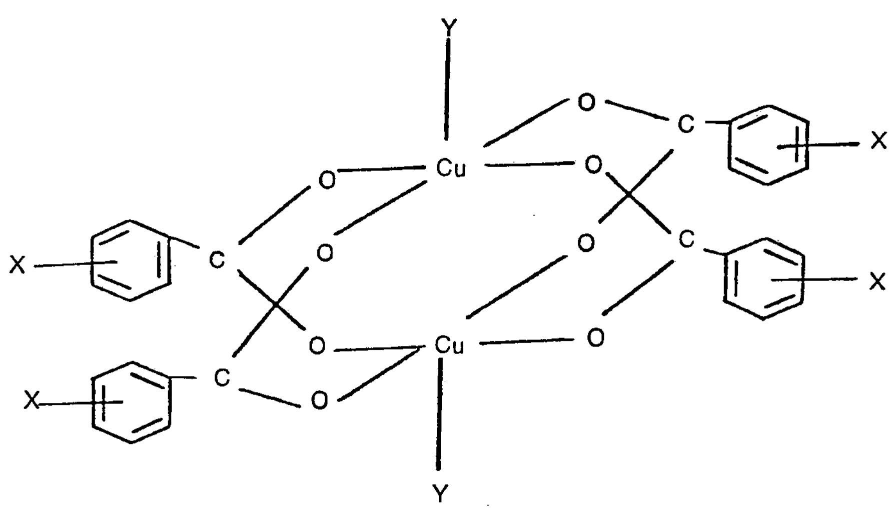

in der Y und X wie in Anspruch 1 definiert sind.

3. Komplexe nach Anspruch 1 oder 2, dadurch gekennzeichnet, daß X OH und Y Dimethylsulfoxid ist.

4. Komplexe nach Anspruch 2, dadurch gekennzeichnet, daß sie die Formel

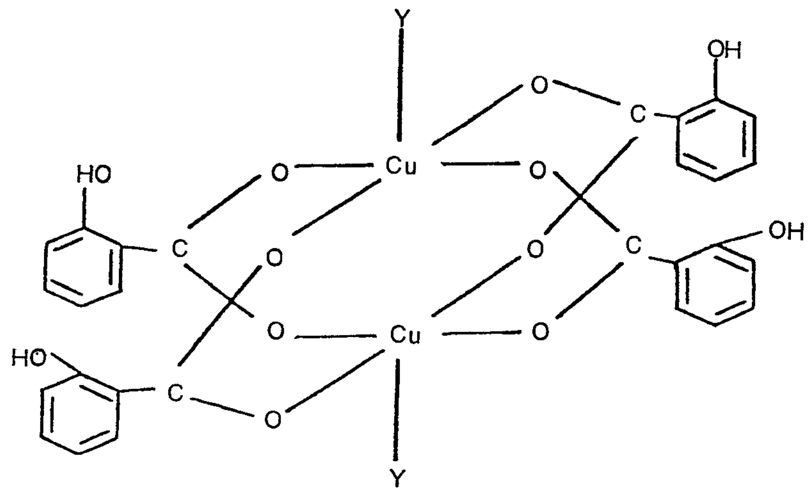

(II)

aufweisen, in der Y wie in Anspruch 2 definiert ist.

5. Verfahren zur Herstellung der Komplexe nach einem der Ansprüche 1—4, dadurch gekennzeichnet, daß es die Umsetzung einer anorganischen Kupfer (II)—Verbindung mit einer substituierten Benzoesäure in Gegenwart von Dimethylsulfoxid oder Dimethylformamid umfaßt.

6. Verfahren nach Anspruch 5, dadurch gekennzeichnet, daß die substituierte Benzoesäure Salicylsäure ist.

7. Verfahren nach Anspruch 5 oder 6, dadurch gekennzeichnet, daß die Kupfer (II)—Verbindung ein Salz oder Kupferhydroxid ist.

8. Pharmazeutische Zusammensetzung, enthaltend einen neutralen Kupferkomplex und einen pharmazeutisch verträglichen Träger, dadurch gekennzeichnet, daß der neutrale Kuperkomplex die Formel

$$Cu[C_6H_4(X)COO]_2\ Y$$

in der Y Dimethylsulfoxid oder Dimethylformamid ist, und in der X an beliebigen Positionen angeordnet sein kann und OH, SH, SeH, $NH_2$ oder

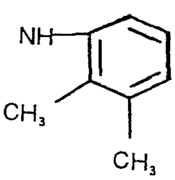

bedeutet, aufweist.

9. Zusammensetzung nach Anspruch 8, dadurch gekennzeichnet, daß der pharmazeutisch verträgliche Träger ein nichtwässriger Träger ist.

10. Zusammensetzung nach Anspruch 8, dadurch gekennzeichnet, daß der Träger Dimethylsulfoxid oder Dimethylformamid; Glycerin und Salicylsäure umfaßt.

11. Zusammensetzung nach einem der Ansprüche 8—10, dadurch gekennzeichnet, daß sie einen Puffer enthält.

12. Zusammensetzung nach einem der Ansprüche 8—11, dadurch gekennzeichnet, daß sie 0,1—15% (Gewicht/Volumen) Wirkstoff enthält.

13. Neutraler Kupferkomplex der Formel

$$Cu(C_6H_4(X)COO]_2\ Y$$

in der Y Dimethylsulfoxid oder Dimethylformamid ist, und in der X an beliebigen Positionen angeordnet sein kann und OH, SH, SeH, $NH_2$ oder

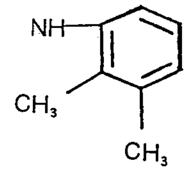

zur Verwendung bei der Behandlung von Entzündungskrankheiten.

**Revendications**

1. Complexes de cuivre neutres, caractérisés en ce qu'ils ont la formule:

$$Cu[C_6H_4(X)COO]_2\ Y$$

dans laquelle Y représente le diméthylsulfoxyle ou la diméthylformamide, et dans laquelle X peut être placé en toute position et est OH, SH, SeH, NH2 ou

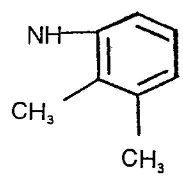

à la condition que, lorsque X est OH, Y n'est pas la diméthylformamide.

2. Complexes selon la revendication 1, caractérisés en ce qu'ils ont la formule:

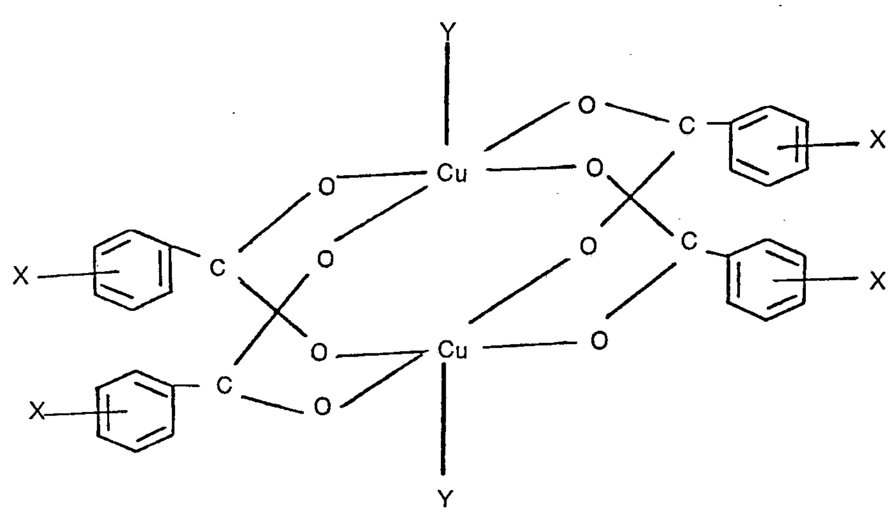

dans laquelle Y et X sont tels que définis dans la revendication 1.

3. Complexes selon la revendication 1 ou la revendication 2, caractérisés en ce que X est OH et Y est le diméthylsulfoxyde.

4. Complexes selon la revendication 2, caractérisés en ce qu'ils ont la formule

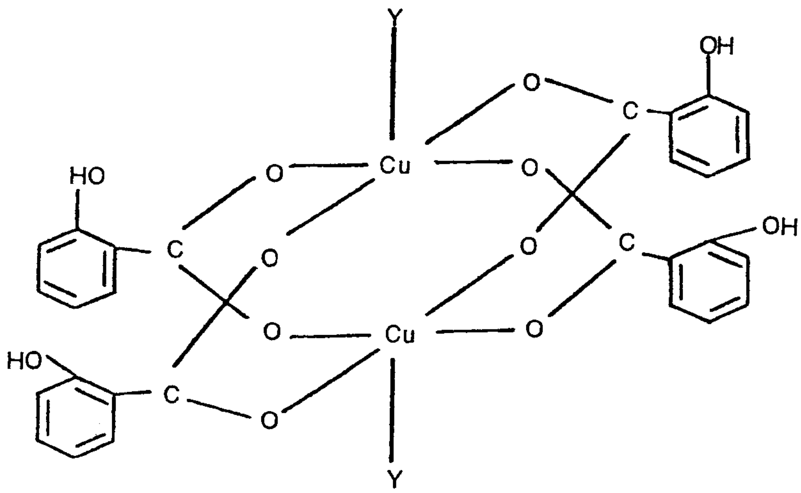

(II)

dans laquelle Y est tel que défini dans la revendication 2.

5. Un procédé de préparation de complexes tels que revendiqués dans l'une quelconqaedes regendications 1 à 4, caractérisé en ce qu'il comporte la réaction d'un composé divalent de cuivre non organique avec un acide benzoïque substitué en présence de diméthylsulfoxyde ou de diméthylformamide.

6. Un procédé selon la revendication 5, caractérisé en ce que l'acide benzoïque substitué est l'acide salicylique.

7. Un procédé selon la revendication 5 ou la revendication 6, caractérisé en ce que le composé de cuivre divalent est un sel ou l'hydroxyde cuivrique.

8. Une composition pharmaceutique comprenant un complexe de cuivre neutre et un support acceptable pharmaceutiquement, caractérisé en ce que ledit complexe de cuivre neutre a la formule:

$$Cu[C_6H_4(X)COO]_2\ Y$$

dans laquelle Y représente le diméthylsulfoxyde ou la diméthylformamide, et dans laquelle X peut être placé en toute position et est OH, SH, SeH, NH2 ou:

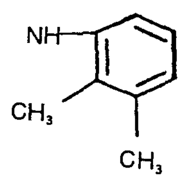

9. Une composition selon la revendication 8, caractérisée en ce que le support acceptable pharmaceutiquement est un support non aqueux.

10. Une composition selon la revendication 8, caractérisée en ce que le support comprend du diméthylsulfoxyde ou de la diméthylformamide; du glycérol; et de l'acide salicylique.

11. Une composition selon l'une quelconque des revendications 8 à 10 contenant un tampon.

12. Une composition selon l'une quelconque des revendications 8 à 11, caractérisée en ce qu'elle contient de 0,1 à 15% en poids/volume d'ingrédient actif.

13. Un complexe de cuivre neutre de formule

$$Cu[C_6H_4(X)COO]_2\ Y$$

dans laquelle Y représente le diméthylsulfoxyde ou la diméthylformamide et dans laquelle X peut être placé en toute position et est OH, SH, SeH, NH2 ou:

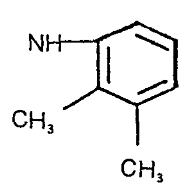

destiné à être utilisé dans le traitement des maladies inflammatoires.